# EUROPEAN PATENT SPECIFICATION

(11) **EP 4 601 608 B1**
(45) Date of publication and mention of the grant of the patent: **11.11.2026**
(21) Application number: 24736165.2
(22) Date of filing: 29.05.2024
(51) Int. Cl.: A61K 8/36, A61K 8/9789, A61K 8/9794, A61Q 15/00

(54) **ANTIPERSPIRANT COMPOSITIONS**
ANTITRANSPIRANT-ZUSAMMENSETZUNGEN
COMPOSITIONS ANTI-TRANSPIRANTES

(30) Priority: 31.05.2023 US 202318203953; 08.08.2023 GB 202312153
(43) Date of publication of application: 20.08.2025
(73) Proprietor: Elementis Specialties, Inc., East Windsor, New Jersey 08512 (US)
(72) Inventor: ORDIALES, Rey, East Windsor, New Jersey 08512 (US); BURCH, Kimberly, East Windsor, New Jersey 08512 (US); CARDARELLI, Victoria, East Windsor, New Jersey 08512 (US); SCHMIDT, Barbara M., East Windsor, New Jersey 08512 (US)
(74) Representative: HGF
(86) International application number: PCT/US2024/031473
(87) International publication number: WO 2024/249514

(56) References cited:
- DE-A1- 102008 053 034
- US-A1- 2004 234 466
- US-A1- 2009 035 228
- US-A1- 2021 378 924
- DATABASE GNPD [online] MINTEL; 8 February 2018 (2018-02-08), ANONYMOUS: "24H Deodorant Roll-On", XP093194675, retrieved from https://www.gnpd.com/sinatra/recordpage/5436019/ Database accession no. 5436019
- DATABASE GNPD [online] MINTEL; 5 January 2023 (2023-01-05), ANONYMOUS: "Nature's Spirit Natural Deodorant Refill", XP093195760, retrieved from https://www.gnpd.com/sinatra/recordpage/10472250/ Database accession no. 10472250
- DATABASE GNPD [online] MINTEL; 7 December 2012 (2012-12-07), ANONYMOUS: "Naturally Harmonising Bath & Shower Gel", XP093195801, retrieved from https://www.gnpd.com/sinatra/recordpage/1937013/ Database accession no. 1937013
- DATABASE GNPD [online] MINTEL; 5 January 2023 (2023-01-05), ANONYMOUS: "Nature's Spirit Natural Deodorant Refill", XP093195760, retrieved from https://www.gnpd.com/sinatra/recordpage/10472250/ Database accession no. 10472250
- DATABASE GNPD [online] MINTEL; 8 February 2018 (2018-02-08), ANONYMOUS: "24H Deodorant Roll-On", XP093194675, retrieved from https://www.gnpd.com/sinatra/recordpage/5436019/ Database accession no. 5436019
- DATABASE GNPD [online] MINTEL; 7 December 2012 (2012-12-07), ANONYMOUS: "Naturally Harmonising Bath & Shower Gel", XP093195801, retrieved from https://www.gnpd.com/sinatra/recordpage/1937013/ Database accession no. 1937013

## Description

### FIELD OF THE INVENTION

The invention is defined in the appended claims and relates to a use of trans-cinnamic acid and to a use of a topical antiperspirant composition.

### BACKGROUND

Antiperspirant compositions are a well-known type of product in the field of personal care and are used to eliminate or reduce body odour, which is important for daily hygiene. These compositions are available in a variety of forms for administration, such as solid sticks, roll-ons, wipes, aerosol sprays and pump sprays.

The compositions generally contain an active ingredient, often referred to as an antiperspirant agent, which may narrow or block the ducts of sweat glands to reduce or prevent perspiration. The antiperspirant agent may also provide a deodorizing effect if it is able to mask odors or it may have antimicrobial activity.

Antiperspirant agents in common use are generally aluminum or aluminum-zirconium salts, such as aluminum chlorohydrate or aluminum zirconium tetrachlorohydrex gly (AZG). These salts dissolve on the skin and plug pores to reduce or prevent sweating.

The search for alternative compounds with similar functionalities have been ongoing to match or improve the sweat reduction properties of aluminum or aluminum zirconium salts.

DE 10 2008 053034 A1 describes the use of hydroxycinnamic acids for inhibiting enzymes, in particular cystathionine beta-lyase, for preventing or treating body odor. XP093195760 is a product leaflet for "Nature's Spirit Natural Deodorant Refill" manufactured by Intiarome. XP093194675 is a product leaflet for "24H Deodorant Roll-On" manufactured by JJSBF. XP093195801 "Naturally Harmonising Bath & Shower Gel" by Monsoon Accessorize. US 2021/378924 A1 describes oral care compositions containing a natural ingredient component. US 2009/035228 A1 describes a skin disinfectant composition comprising one or more essential oil and one or more fruit acid. US 2004/234466 A1 describes a non-therapeutic method for the inhibition of beta-glucuronidase.

### SUMMARY OF THE INVENTION

The invention is defined in the appended claims.

The invention relates to the use of trans-cinnamic acid as an antiperspirant agent for reducing or preventing perspiration. In a further aspect of the disclosure, a topical antiperspirant composition comprises the antiperspirant agent, which consists of *trans-*cinnamic acid. The topical antiperspirant composition comprises:
(a) the antiperspirant agent in a total amount of 1.5 to 25.0 wt%, and
(b) a topically acceptable carrier; and
wherein the antiperspirant composition is in the form of a cream, a gel, a paste, a liquid, a lotion, a foam, a scrub or a powder.

Also described is an antiperspirant composition comprising an antiperspirant agent, wherein the antiperspirant agent comprises a phytochemical, which is an optionally substituted benzenepropenoic acid.

In an aspect of the disclosure, an antiperspirant composition is described. The antiperspirant composition comprises either: (a) a natural extract or (b) an antiperspirant agent obtained or obtainable from the natural extract.

The natural extract in the second aspect is cinnamon extract or is cinnamon extract and *Emblica officinalis* extract. It is preferred that the natural extract is cinnamon extract.

The antiperspirant agent in (b) may be present in the antiperspirant composition in the form of the natural extract in (a).

It has surprisingly been found that certain classes of phytochemicals or plant extracts possess antiperspirant properties. These naturally derivable materials can be used as antiperspirant agents, particularly in personal care products. They can provide a comparable antiperspirant effect to the aluminum or aluminum-zirconium salts that are typically included in most commercially available antiperspirant products. Thus, the phytochemicals or plant extracts can be included as antiperspirant agents instead of the aluminum or aluminum-zirconium salts. These materials, particularly the natural extracts, may also impart a pleasant odor to the antiperspirant composition. There is also no concern over risks to health associated with these ingredients.

In a further aspect of the disclosure, a method of method of reducing or preventing perspiration is described herein. The method comprises the step of applying a topical antiperspirant composition to skin, wherein the topical antiperspirant composition comprises:
(c) an antiperspirant agent in a total amount of 1'.5 to 25.0 wt%, and
(d) a topically acceptable carrier;
wherein the antiperspirant agent consists of trans-cinnamic acid, and wherein the antiperspirant composition is in the form of a cream, a gel, a paste, a liquid, a lotion, a foam, a scrub or a powder.

Also described is the use of a phytochemical as an antiperspirant agent for reducing or preventing perspiration, wherein the phytochemical is an optionally substituted benzenepropenoic acid.

A further aspect of the disclosure relates to the use of a natural extract as an antiperspirant agent for reducing or preventing perspiration. The natural extract is cinnamon extract or is cinnamon extract and *Emblica officinalis* extract. It is preferred that the natural extract is cinnamon extract.

### DETAILED DESCRIPTION

The following definitions apply to the disclosure herein, including all aspects of the invention and their embodiments.

The term "personal care" as used herein, whether used alone or in conjunction with another term, refers to a non-therapeutic use, such as a non-therapeutic, cosmetic use. This term typically refers to use for personal hygiene.

The compounds and intermediates described herein may be named according to the IUPAC (International Union for Pure and Applied Chemistry) or the CAS (Chemical Abstracts Service) nomenclature systems or by their commercial trade names. It should also be understood that any reference to a compound described herein, such as a polyurea, a primary monoamine, a diamine or a triamine, encompasses all stereoisomers (e.g. cis and trans isomers) and/or optical isomers (e.g. R and S enantiomers) of such compounds, in substantially pure form and/or any mixtures of the foregoing in any ratio.

The term "phytochemical" as used herein refers to a chemical compound that is produced by a plant (e.g. through primary or secondary metabolism). In other words, a phytochemical is a naturally occurring, biologically active compound found in a plant. The phytochemical typically has biological activity that protects the plant from the environment, disease or a predator.

The term "flavonoid" as used herein refers to a compound having a 15-carbon core structure (e.g. skeleton) consisting of two phenyl rings (referred to as the "A" and "B" rings) and a heterocyclic ring (referred to as the "C" ring), which contains an oxygen atom. The term "flavonoid" is used in the conventional, restricted sense as referring only to those compounds with a C₆-C₃-C₆ carbon framework (e.g. core structure or skeleton) having the structure of a chromane or a 1-benzopyran (chromene), in which the fused benzene ring is designated as ring A and the 3,4-dihydro-2*H*-pyran or the pyran as ring C, along with a phenyl group (ring B) on ring C (see, for example, the "Nomenclature of flavonoids" by A. P. Rauter *et al.* - Reference 1). The general structure of a flavonoid with ring labelling is shown below.

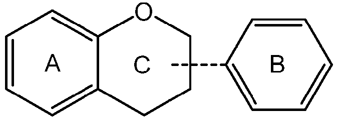

Depending on the position of the linkage of ring B to the chromane/1-benzopyran (chromene) moiety, the term "flavonoid" comprises, or consists of, the sub-classes of compounds known as 2-flavonoids, 3-flavonoids (e.g. also known as isoflavonoids) and 4-flavonoids (e.g. also known as neoflavonoids).

The term "2-flavonoid" as used herein refers to a compound having a core structure or skeleton as represented by formula (A-1).

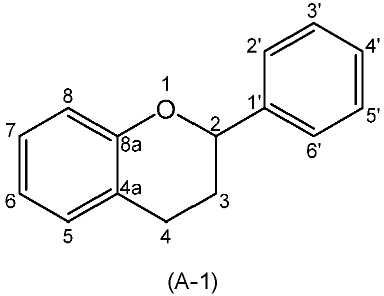

In formula (A-1), the atoms of the core structure are numbered in accordance with the IUPAC numbering scheme for this sub-class of compounds.

For the avoidance of doubt, formula (A-1) represents the core structure or skeleton of a 2-flavonoid. Rings A to C of the core structure may include substituents. Ring C may include a carbon-carbon double bond (in addition to the unsaturation arising from the fused phenyl group, which is ring A) and/or a carbonyl group.

In general, the term "2-flavonoid" includes a flavan, a flavone or a flavanol.

When ring C in formula (A-1) does not include a carbon-carbon double bond or a carbonyl group, then formula (A-1) represents a flavan. The term "flavan" refers to a compound with a 2-phenyl-3,4-dihydro-2*H*-1-benzopyran skeleton.

When ring C in formula (A-1) is substituted with a hydroxy group, such as at the 3-position and/or the 4-position of ring C, then the 2-flavonoid is a flavanol.

The core structure or skeleton of the flavanol may be a flavan-3-ol (as represented by formula (A-2)), a flavan-4-ol (as represented by formula (A-3)) or a flavan-3,4-diol (as represented by formula (A-4)).

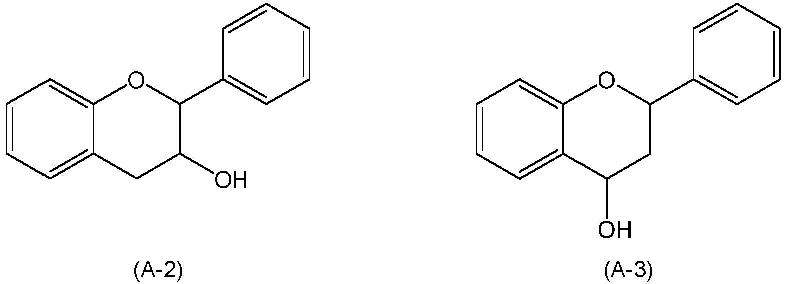

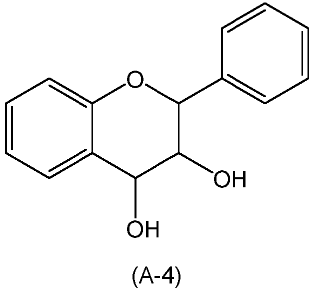

When ring C in formula (A-1) includes a carbonyl group, such that the compound is derived from a 2-phenyl-2,3-dihydro-4*H*-1-benzopyran-4-one skeleton, then the 2-flavonoid is a flavanone, specifically a flavan-4-one (e.g. as represented by formula (A-5)).

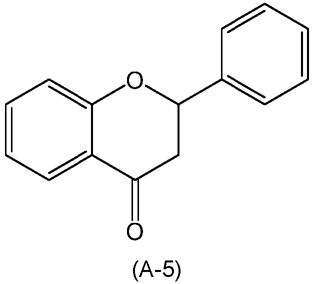

It is preferred that the core structure or skeleton of the 2-flavonoid is not an anthocyanidin (also known as an anthocyanin). An anthocyanidin is a compound derived from a flavylium (2-phenyl-1λ⁴-benzopyran-1-ylium or 2-phenylchromenylium) ion.

The term "glucogallin" as used herein refers to the following compound. 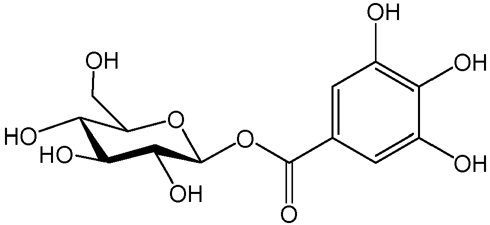 Glucogallin is typically present in a natural extract of *Emblica officinalis.* Thus, glucogallin may be obtained or is obtainable from *Emblica officinalis* extract.

The term "saberry" or "saberry extract" as used herein is synonymous with *Emblica officinalis* or *Emblica officinalis* extract, respectively, and refers to fruits or an extract of fruits of *Emblica officinalis* (synonymous with *phyllanthus emblica*), which is more commonly known as amla berry or Indian gooseberry.

As used herein by itself or in conjunction with another term or terms, "substituted" indicates that a hydrogen atom on a molecule has been replaced with a different atom or group of atoms. The atom or group of atoms replacing the hydrogen atom is a "substituent." It should be understood that the terms "substituent", "substituents", "moiety", "moieties", "group", or "groups" refer to substituent(s).

The various hydrocarbon-containing moieties provided herein may be described using a prefix designating the minimum and maximum number of carbon atoms in the moiety, namely "Cₐ-C_{b}". For example, Cₐ-C_{b} alkyl indicates an alkyl moiety having the integer "a" to the integer "b" number of carbon atoms, inclusive.

As used herein, the term "alkyl", whether used alone or in conjunction with another term, refers to a branched or unbranched saturated hydrocarbon chain, unless specified otherwise. An alkyl group is typically unsubstituted, unless indicated otherwise. An alkyl group may have 1 to 6 carbon atoms, such as 1 to 4 carbon atoms. Representative examples include, but are not limited to, methyl, ethyl, n-propyl, i-propyl, n-butyl, i-butyl, s-butyl, t-butyl, n-pentyl, n-hexyl.

As used herein, the term "alkoxy" refers to an alkyl-oxy group, wherein the alkyl group is as previously defined. An alkoxy group is typically unsubstituted, unless specified otherwise. Representative examples include -OMe, -OEt, -OⁿPr, -OⁱPr.

The term "deodorant" as used herein refers to an agent, material or composition that masks or inhibits the formation of unpleasant body odors.

Amounts of a material, such as a compound, composition or an ingredient, are typically defined in terms of the wt% of the composition.

The term "about" when used herein in conjunction with a measurable value encompasses reasonable variations of the value, for instance, to allow for experimental error in the measurement of said value.

As used in the present disclosure, the term "comprises" has an open meaning, which allows other, unspecified features to be present. This term embraces, but is not limited to, the semi-closed term "consisting essentially of" and the closed term "consisting of". Unless the context indicates otherwise, the term "comprises" may be replaced with either "consisting essentially of" or "consists of". The term "consisting essentially of" may also be replaced with "consists of".

For the avoidance of doubt, the expression "consists essentially of" as used herein limits the scope of a feature to include the specified materials, and any other materials or steps that do not materially affect the basic and novel characteristics of that feature, such as, for example, minor impurities. The expression "consists essentially of' embraces the expression "consisting of".

### Antiperspirant agent

The invention relates to the use of products that are naturally derived or obtainable as antiperspirant agents.

The antiperspirant agent is for reducing or preventing perspiration. Thus, the antiperspirant agent is an active ingredient for reducing or preventing perspiration in the antiperspirant composition of the invention.

Perspiration is a physiological body function that helps to control and preserve body temperature at approximately 37°C. Sweat is produced by sweat glands, which are cutaneous annexes composed by an outer layer of myoepithelial contractible cells and an inner layer of secreting cells. When the body temperature increases, the sweat glands are activated by a signal sent by the brain to produce sweat. Besides preserving stability of body temperature, sweat also humidifies skin and releases toxins. Excess transpiration can cause discomfort, such as underarm bad odor and lesions.

Antiperspirants work by limiting the quantity of sweat released by the skin through the ducts of sweat glands by forming a buffer.

The antiperspirant agent of the invention is for external use (e.g. on the human body).

The antiperspirant composition or the antiperspirant agent is for application to a surface of a body, particularly the skin.

The antiperspirant agent consists of *trans*-cinnamic acid.

For all aspects of the invention, the antiperspirant agent will now be further described by way of the following numbered paragraphs:
1. The antiperspirant agent consists of *trans*-cinnamic acid.
   Trans-cinnamic acid may be found in some cinnamon extracts.
2. The antiperspirant agent in paragraph 1 obtained or obtainable from a natural extract.
3. The antiperspirant agent in paragraph 2, wherein the natural extract is cinnamon extract.
4. The antiperspirant agent in any of paragraphs 1 to 3, which does not include an aluminum chlorohydrate or aluminum zirconium tetrachlorohydrex gly.
5. The antiperspirant agent in any of paragraphs 1 to 4, which does not include an aluminum-containing antiperspirant agent, preferably does not include aluminum.
6. The antiperspirant agent in paragraph 5, which does not include zirconium.
7. The antiperspirant agent in any one of paragraphs 1 to 6, wherein the antiperspirant agent is an anti-oxidant.
8. The antiperspirant agent in any one of paragraphs 1 to 7, wherein the antiperspirant agent is a protein coagulant.
9. The antiperspirant agent in any one of paragraphs 1 to 8, wherein the antiperspirant agent is a preservative.
10. The antiperspirant agent in any one of paragraphs 1 to 9, wherein the antiperspirant agent is anti-bacterial agent.

The antiperspirant agent in any one of paragraphs 1 to 10 above may be present in the antiperspirant composition in the form of the natural extract in an aspect of the invention.

For all aspects of the invention, the natural extract will now be further described by way of the following numbered paragraphs:
11. The natural extract is cinnamon extract (e.g. only cinnamon extract).
12. The natural extract in paragraph 11, wherein the cinnamon extract comprises one or more 2-flavonoids.
13. The natural extract in paragraph 12, wherein at least one of the one or more 2-flavonoids is a flavanol, preferably a flavan-3-ol.
14. The natural extract in paragraph 13, wherein the flavanol or the flavan-3-ol is afzelechin, epiafzelechin, catechin, epicatechin, gallocatechin, epigallocatechin or a combination of two or more thereof.
15. The natural extract in any of paragraphs 12 to 14, wherein at least one of the one or more 2-flavonoids is a flavanone, preferably a flavan-4-one.
16. The natural extract in paragraph 15, wherein the flavanone or the flavan-4-one is rutin.
17. The natural extract in any of paragraphs 11 to 16, wherein the cinnamon extract comprises *trans*-cinnamic acid.
18. The natural extract in any one of paragraphs 11 to 17, wherein the natural extract is an anti-oxidant.
19. The natural extract antiperspirant agent in any one of paragraphs 11 to 18, wherein the natural extract is a protein coagulant.
20. The natural extract in any one of paragraphs 11 to 19, wherein the natural extract is a preservative.
21. The natural extract in any one of paragraphs 11 to 20, wherein the natural extract is anti-bacterial agent.

Without wishing to be bound by theory, the antiperspirant agents of the invention, whether present as individual compounds or in the form of a natural extract, may plug the pores of sweat glands by causing protein aggregation and/or by binding to specific receptors in the wall of the sweat duct.

It has surprisingly been found that cinnamic acid has an antiperspirant effect. It is believed that this compound can cause coagulation or agglomeration of proteins.

Typically, the antiperspirant agent and/or natural extract has at least a 20% sweat reduction in at least 50% subjects tested, as measured according to US FDA Guidelines (Reference 3). The term "subjects tested" is synonymous with the "target population" mentioned in the US FDA Guidelines.

In general, the antiperspirant agent and/or the natural extract may also be a deodorizing agent (e.g. a deodorant).

Additionally or alternatively, the antiperspirant agent and/or the natural extract may have an astringent effect, a toning effect and/or an anti-aging effect on skin, preferably an astringent effect.

### Antiperspirant composition

The antiperspirant composition comprises an antiperspirant agent as defined in the headed section above, including as defined in any of paragraphs 1 to 10. The antiperspirant agent may be present as a natural extract as defined above, including as defined in any of paragraphs 11 to 21. The antiperspirant agent may be obtained or obtainable from the natural extract.

Typically, the antiperspirant agent as defined above is the sole antiperspirant agent in the antiperspirant composition.

When the antiperspirant composition comprises a natural extract as defined above, then it is preferred that the natural extract is the sole antiperspirant agent.

In general, the antiperspirant composition does not comprise aluminum chlorohydrate or aluminum zirconium tetrachlorohydrex gly. It is preferred that the antiperspirant composition does not comprise an aluminum-containing antiperspirant agent, more preferably the antiperspirant composition does not comprise aluminum and optionally zirconium.

Additionally or alternatively, the antiperspirant composition does not comprise 4-hydroxybenzaldehyde. It is preferred that the antiperspirant composition does not comprise (i) a benzaldehyde or a benzaldehyde-derivative compound of formula I: 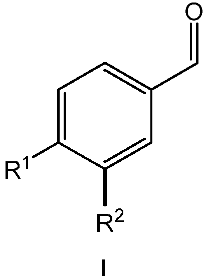 wherein R' is a residue selected from the group consisting of H, CH₂, CH₃, OH, and OCH₃, wherein R² is a residue selected from the group consisting of H, OH, OCH₃, and OCH₂O, and wherein if R² is OCH₂O, then R' is CH₂ and/or forms a bond with R²; and/or (ii) benzaldehyde, 4-methylbenzaldehyde, heliotropine, vanillin, 4-hydroxybenzaldehyde, 3-hydroxybenzaldehyde, 4-methoxybenzaldehyde, 3-methoxybenzaldehyde, or 3-hydroxy-4-methoxybenzaldehyde (isovanillin).

The antiperspirant composition comprises an effective amount of the antiperspirant agent or the natural extract. The effective amount is an amount that will reduce or prevent perspiration.

The topical antiperspirant composition of the invention comprises the antiperspirant agent in a total amount of 1.5 to 25.0 wt%.

In other aspects, the antiperspirant composition typically comprises a total amount of from about 0.1 to about 25.0 wt% of the antiperspirant agent, preferably from about 0.5 to about 15.0 wt%, more preferably from about 1.0 to about 10.0 wt%, such as from about 1.0 to about 5.0 wt% of the antiperspirant agent.

The antiperspirant composition preferably comprises a total amount of about 0.1 to about 4.0 wt% of the antiperspirant agent, such as about 0.5 to about 4.0 wt%, more preferably from about 1.0 to 4.0 wt%, such as about 1.0 to about 3.5 wt% of the antiperspirant agent.

When the antiperspirant agent consists of *trans*-cinnamic acid, then the antiperspirant composition comprises the *trans*-cinnamic acid in a total amount of about 1.5 to about 25.0 wt%, preferably from about 1.0 to about 20.0 wt% (e.g. about 3.0 to about 20.0 wt%), more preferably from about 1.5 to about 15.0 wt% (e.g. about 5.0 to 15.0 wt%), such as from about 2.0 to about 10.0 wt%.

The antiperspirant composition typically comprises a total amount of from about 5.0 to about 40.0 wt% of the natural extract, preferably from about 10.0 to about 30.0 wt%, more preferably from about 15.0 to about 25.0 wt% of the natural extract.

The amount of natural extract that is included in the antiperspirant composition will depend on the composition of the extract, particularly the type and amount of ingredients that are present in the natural extract.

In general, the antiperspirant composition is a cosmetic antiperspirant composition, particularly a cosmetic antiperspirant composition for personal care.

The antiperspirant composition is for topical use. Thus, the antiperspirant composition is a topical antiperspirant composition.

The antiperspirant composition is for topical application to skin, preferably a surface of the skin. The antiperspirant composition may be formulated for a specific mode of topical application.

Typically, the antiperspirant composition comprises a topically acceptable carrier. The topically acceptable carrier will depend on the type of formulation and its mode of topical application.

The topically acceptable carrier may be water, an alcohol, an ether, an oil or a wax.

The topically acceptable carrier may be water or water and oil, such as when the antiperspirant composition is in the form of a gel, a paste, a liquid, a lotion or a foam.

The topically acceptable carrier may be an alcohol. The alcohol may be a mono-alcohol or a polyhydric alcohol (e.g. an alcoholic compound having two or more hydroxy groups).

When the alcohol is a mono-alcohol, then then mono-alcohol may be ethanol or a fatty alcohol. The fatty alcohol may have a straight chain length of 6 to 18 carbon atoms. It is preferred that the fatty alcohol is stearyl alcohol.

When the alcohol is a polyhydric alcohol, then the polyhydric alcohol may be a polymeric alcohol or a monomeric alcohol. The polymeric alcohol may be a polyalkylene glycol, such as PEG or PPG.). The monomeric alcohol may be ethylene glycol, propylene glycol, glycerol or a sugar, such as glucose, sorbitol or xylitol.

The topically acceptable carrier may be an ether. The ether may be PPG-14 butyl ether.

The topically acceptable carrier may be an oil. The oil may be hydrogenated castor oil.

The antiperspirant composition may be provided as, or included in, a deodorant, such as a body deodorant for personal hygiene purposes.

The antiperspirant composition is in the form of a cream, a gel, a paste, a liquid, a lotion, a foam, a scrub or a powder.

Typically, the antiperspirant composition is a leave-on composition. A leave on composition may be applied to skin, or a surface of the skin, and left on for a period of time (e.g. from 1 minute to 24 hours), after which it may be wiped or rinsed off with water, usually during the regular course of washing.

When the antiperspirant composition is in the form of a liquid, then the liquid may be a water-in-oil emulsion or an oil-in-water emulsion.

In general, when the antiperspirant composition is in the form of a liquid, then the liquid may be a sprayable liquid.

The antiperspirant composition may be for delivery as an aerosol. The topically acceptable carrier may comprise a propellant.

The antiperspirant composition may comprise a lubricant. The lubricant may, for example, be a cyclomethicone, a dimethicone, a phenyltrimethicone or an ethyl methicone. It is preferred that the lubricant is a cyclomethicone and/or phenyltrimethicone.

The antiperspirant composition of the invention may further comprise a natural oil. The natural oil may be included for skin care benefits.

Typically, the natural oil comprises an unsaturated fat, such as a polyunsaturated fat (e.g. a triglyceride derived from linoleic acid) and/or a monounsaturated fat (e.g. a triglyceride derived from oleic acid).

The antiperspirant composition may further comprise an antioxidant ingredient. An antioxidant ingredient may be included to chemically stabilise the antiperspirant or any other natural ingredient that may be present, such a natural oil, particularly a natural oil comprising a polyunsaturated fat and/or a monounsaturated fat.

The antioxidant ingredient may be selected from ascorbic acid, butyl hydroxy toluene, pentaerythrityl tetra-di-t-butyl hydroxyhydrocinnamate and tris-(tetramethylhydroxypiperidinol) citrate.

The antiperspirant composition may include a fragrance.

The antiperspirant composition may include a thickener. The thickener may be fumed silica or a cellulose ether, such as hydroxypropyl cellulose, hydroxypropyl methyl cellulose, hydroxyethyl cellulose, carboxymethyl cellulose, cetyl hydroxyethyl cellulose, hydroxybutyl methyl cellulose, methyl hydroxyethyl cellulose or xanthan gum. It is preferred that the thickener is fumed silica.

The antiperspirant composition may include a surfactant. The surfactant may be an anionic surfactant, a cationic surfactant, a non-ionic surfactant or an amphoteric surfactant.

The antiperspirant composition may include a personal care ingredient. The personal care ingredient may be a skin care agent, such as an emollient, a humectant, a skin barrier promoter or a skin appearance modifier.

The antiperspirant composition may comprise a wide range of other optional components, such as a binder, a biological additive, a buffering agent, a colorant, a fragrance, a conditioner, an exfoliating agent, a pH adjuster, a preservative, a skin soothing agent or a skin healing agent.

It is preferred that the antiperspirant composition comprises a preservative and optionally one or more components selected from a binder, a biological additive, a buffering agent, a colorant, a fragrance, a conditioner, an exfoliating agent, a pH adjuster, a skin soothing agent and a skin healing agent.

Typically, the antiperspirant composition has at least a 20% sweat reduction in at least 50% subjects tested, as measured according to US FDA Guidelines (Reference 3).

In general, the antiperspirant composition may also be a deodorant composition.

The antiperspirant composition can be manufactured by conventional methods known in the art.

### Methods and Uses

The uses of the invention and the methods described herein are non-therapeutic.

The antiperspirant composition is for use as a cosmetic, particularly for personal care.

The antiperspirant composition or the antiperspirant agent is applied to skin, preferably topically applied to skin.

The antiperspirant composition or the antiperspirant agent is applied to a surface of the skin.

In general, the skin is of a mammal, preferably a human.

Typically, the antiperspirant composition or the antiperspirant agent is applied to an area of skin where perspiration occurs, preferably an area of the skin where there are sweat glands, such as apocrine sweat glands or eccrine sweat glands, preferably apocrine sweat glands.

The area of skin may be on a thigh (e.g. inner thigh), the groin, the back, the upper lip, an armpit, the forehead, the knee (e.g. the back of the knee), the neck (e.g. back of the neck), the buttock, the chest, the scalp or the hand. It is preferred that the area of skin is an armpit.

The antiperspirant composition or the antiperspirant agent may be applied by a direct body contact applicator, such as a roll-on lotion or a solid stick product.

Alternatively, the antiperspirant composition or the antiperspirant agent may be applied by a non-contact applicator, such as by spraying onto the skin. The non-contact applicator may be an aerosol spray, a pump spray or a squeeze spray.

The antiperspirant agent, the natural extract and/or the antiperspirant composition may be for use as a deodorant.

### Examples

The invention will now be illustrated by the following non-limiting examples.

### Examples 1 to 3

Formulation examples 1 to 3 shown in Table 1 were prepared by conventional methods.

**Table 1**

| Ingredient | Formulation (F) No. | | |
|---|---|---|---|
| | 1 | 2 | 3 |
| Natural extract | E1 | E1+E2 | E3 |
| Cyclomethicone | ✔ | ✔ | ✔ |
| Aluminum starch succinate | ✔ | ✔ | ✔ |
| Phenyltrimethicone | ✔ | ✔ | ✔ |
| PPG-14 butyl ether | ✔ | ✔ | ✔ |
| Fumed silica | ✔ | ✔ | ✔ |
| Hydrogenated castor oil | ✔ | ✔ | ✔ |
| Stearyl alcohol | ✔ | ✔ | ✔ |
| 3-phenoxyethanol-1,2-propanediol | ✔ | ✔ | ✔ |

In Table 1 above, E1 is cinnamon extract; E2 is *Emblica officinalis* extract (not in accordance with the invention); and E3 is trans-cinnamic acid. The extracts were obtained from Sabinsa Corporation. Each natural extract was included in an amount in each formulation to provide the same amount of antiperspirant agent across the formulations.

### Comparative Examples

The formulations shown in Table 2 were prepared to provide a comparison to the formulations in Table 1. No natural extracts were included in these formulations.

**Table 2**

| Ingredient | Comparative Formulation (CF) No. | |
|---|---|---|
| | 1 | 2 |
| Natural extract | - | - |
| Antiperspirant | AP1 | AP2 |
| Cyclomethicone | ✔ | ✔ |
| Aluminum starch octenylsuccinate | ✔ | ✔ |
| Phenyltrimethicone | ✔ | ✔ |
| PPG-14 butyl ether | ✔ | ✔ |
| Fumed silica | ✔ | ✔ |
| Hydrogenated castor oil | ✔ | ✔ |
| Stearyl alcohol | ✔ | ✔ |

In Table 2 above, AP1 is aluminum chlorohydrate and AP2 is aluminum zirconium tetrachlorohydrate glycine (AZG).

### Roll-on antiperspirant product

The method of manufacturing a roll-on product is set out below.
1. Mix cyclomethicone, phenyltrimethicone, stearyl alcohol, PPG-14 butyl ether and the hydrogenated castor oil.
2. Heat to 82°C to melt the solids.
3. While maintaining a temperature of 82°C, slowly add silica using a high-speed disk type dispenser.
4. Once all silica is added, increase mixing speed so that peripheral velocity is almost 4000 ft/minute or higher for satisfactory dispersion.
5. Add starch followed by the Natural AP powder.
6. It is important to disperse each component uniformly before adding the next.
7. Once all components are added, cool the mixture to 55°C.

### In Vivo Test Procedure

### Study Design

The formulation examples were tested in a standard hot room assessment clinical study. The study panellists underwent thermal stress after application of the antiperspirant compositions. The sweat reduction was determined by gravimetric collection of the sweat.
Subjects: 40 female panellists.
Conditions: 17-day washout period in which the panellists did not use any antiperspirants.
Test Design: Subjects underwent 5-day application period where each was given 400 mg + 50 mg of one of the formulation examples. After the 5^{th} day, sweat collection was conducted after the panellists entered the hot room (37.8°C ± 1.1°C [100°F ± 2°F] and 35% ± 5% relative humidity) for 80 minutes to induce sweating.
Analysis of Data: The data were transformed to their natural logarithms and the averages were analyzed according to the method described by Murphy and Levine (Reference 2). The analysis of covariance model included factors for sequence, subjects within a sequence, test articles, axilla and baseline as the covariate.

For each formulation, the %Change in sweat reduction was determined. The clinical study data showed that all of the formulations were effective at reducing sweat, with formulations F1 to F3 showing a sweat reduction greater than 30% when compared to baseline. A sweat reduction of at least 20% is considered to be suitable for commercial use as an antiperspirant agent.

Formulation F3 showed sweat efficacy results that are parity results when compared to the comparative products with aluminum or aluminum zirconium.

### Examples 4 and 5

Further experiments were performed using Formulation No. 3 (see Table 1 above), except that the amount of trans-cinnamic acid was varied. Formulation No. 4 contained 3 wt% of trans-cinnamic acid. Formulation No. 5 contained 10 wt% of trans-cinnamic acid. These formulations were prepared using the same method for manufacturing roll-on antiperspirant products given above.

### In vivo tests

In vivo tests were performed using each formulation.

For inclusion in the studies, participants needed to show an amount of sweat of 150 mg in each armpit in an initial assessment. The same methodology as set out above was used to perform these tests, except for the following details.

Formulation No. 4: 30 panellists participated in the study and during the 5-day application period, each panellist was given 400 mg of the formulation.

Formulation No. 5: 33 panellists participated in the study and were subjected to an 18-day washout period. Each panellist was given 400 mg of the formulation.

When in the study there were pre-treatment records (baseline) in the evaluation of armpit sweating after the last application, the ratio of the treated armpit with the investigational product adjusted to the ratio of the right-to-left armpit sweating rate is defined for each individual by the formula: $Z = \left(PC\times T\right) / \left(PT\times C\right)$ where Z is the adjusted ratio from left to right; PC is the amount of sweat obtained in the pre-treatment for the control armpit; PT is the amount of sweat obtained in the pre-treatment for the armpit that will receive the treatment; T is the amount of sweat obtained for the armpit with the formulation of the invention; and C is the amount of sweat obtained for the control armpit.

The results are shown in Tables 3 and 4 below.

**Table 3: Formulation No. 4**

| Sweating Values, 8 hours | | | | | | |
|---|---|---|---|---|---|---|
| | Armpit pre-treatment to be treated (PT) (mg) | Armpit pre-treatment control (PC) (mg) | Treated Armpit (T) (mg) | Control Armpit (C) (mg) | Z | Z - 0.8 |
| Mean | 1832.7 | 1739.3 | 943.3 | 2227.0 | 0.37 | -0.43 |
| Median | 1790.0 | 1640.0 | 775.0 | 2035.0 | 0.32 | -0.48 |
| Minimum | 1310.0 | 1010.0 | 400.0 | 1630.0 | 0.16 | -0.64 |
| Maximum | 2720.0 | 2950.0 | 4920.0 | 4730.0 | 1.00 | 0.20 |
| Standard error | 68.7 | 86.5 | 150.3 | 105.8 | 0.03 | 0.03 |
| IC 95% | 140.6 | 176.9 | 307.4 | 216.5 | 0.06 | 0.06 |
| % of research participants with reduction greater than 20% | | | | | | **96.67** |
| *P-Value | | | | | | 0.000 |

**Table 4: Formulation No. 5**

| Sweating Values, 8 hours | | | | | | |
|---|---|---|---|---|---|---|
| | Armpit pre-treatment to be treated (PT) (mg) | Armpit pre-treatment control (PC) (mg) | Treated Armpit (T) (mg) | Control Armpit (C) (mg) | Z | Z - 0.8 |
| Mean | 1849.7 | 1817.5 | 665.8 | 1994.2 | 0.33 | -0.47 |
| Median | 1810.0 | 1850.0 | 650.0 | 2050.0 | 0.34 | -0.46 |
| Minimum | 1200.0 | 1150.0 | 410.0 | 1260.0 | 0.17 | -0.63 |
| Maximum | 3000.0 | 2790.0 | 1090.0 | 2750.0 | 0.49 | -0.31 |
| Standard error | 58.1 | 59.7 | 28.7 | 57.7 | 0.01 | 0.01 |
| IC 95% | 118.4 | 121.7 | 58.5 | 117.6 | 0.03 | 0.03 |
| % of research participants with reduction greater than 20% | | | | | | **100.0** |
| *P-Value | | | | | | 0.000 |

In both studies, no statistically significant differences were found for the median values of sweating between the product and the control. According to the methodology used to evaluate the antiperspirant effect, Formulation Nos. 4 and 5 showed a reduction in sweating 8 hours after the last application. Both tests showed that at least 50% of the research participants had a reduction in sweating of at least 20%.

### Example 6

A study to assess the deodorant effect was performed using Formulation No. 5. The deodorancy study was designed to be a single center, single blind, randomized, trial to determine the deodorancy effectiveness of trans-cinnamic acid.

In this study, 12 panellists were pre-conditioned by using only soap and abstaining from the use of deodorants, antiperspirants, perfumed or medicated products for a minimum of 48 hrs, as in the studies described above.

On the test day, the panellists were evaluated for odor by 3 judges. Odor scores were measured from 0 (no odor) to 4 (severe). To participate in the study, the panellists were required to have an odor score of at least 3.

Immediately after the evaluation and qualification of the panellists, Formulation No. 5 was applied to one axilla. Randomized application was conducted for all panellists. The panellists were then evaluated for odor at 8 hr, 24 hr and 48 hr time points.

Notably, the results from the study showed that there was a statistically significant decrease in odor scores at 8hr, 24 hr and 48 hr time points.

### References

The following documents are referenced in the description.
1. A. P. Rauter et al.; "Nomenclature of flavonoids (IUPAC Recommendations 2017)"; Pure Appl. Chem., 90(9), (2018), 1429-1486.
2. T. D. Murphy & M. J. Levine; "Analysis of Antiperspirant Efficacy Test Results"; Journal of the Society of Cosmetic Chemists, 42, (1991), 167-197.
3. "Guidelines for Effectiveness Testing of OTC Antiperspirant Drug Products", published by the US FDA, when read in conjunction with § 350.60 (21 CFR 350.60) of the final monograph (final rule) for OTC antiperspirant drug products, published in the US FEDERAL REGISTER on June 9, 2003 (68 FR 34273).

All headings and sub-headings are used herein for convenience only and should not be construed as limiting the invention in any way.

The use of any and all examples, or exemplary language (e.g. "such as") provided herein, is intended merely to better illuminate the invention and does not pose a limitation on the scope of the invention unless otherwise paragraphed. No language in the specification should be construed as indicating any non-paragraphed element as essential to the practice of the invention.

## Claims

1. Use of trans-cinnamic acid as an antiperspirant agent for reducing or preventing perspiration.

2. Use according to claim 1 as a cosmetic for personal care.

3. Use according to claim 1 or claim 2 for topical application to skin.

4. Use according to any one of claims 1 to 3, for application to an area of skin having sweat glands.

5. Use according to any one of claims 1 to 4, wherein the trans-cinnamic acid is used as a an antiperspirant agent and as a deodorant.

6. Use according to any one of claims 1 to 5, wherein a topical antiperspirant composition comprises the antiperspirant agent, wherein the topical antiperspirant composition comprises:
(a) the antiperspirant agent in a total amount of 1.5 to 25.0 wt%, and
(b) a topically acceptable carrier; and
wherein the antiperspirant composition is in the form of a cream, a gel, a paste, a liquid, a lotion, a foam, a scrub or a powder.

7. Use according to claim 6, wherein the topical antiperspirant composition comprises the trans-cinnamic acid in a total amount of 3.0 to 20.0 wt%.

8. Use according to claim 6, wherein the topical antiperspirant composition comprises the trans-cinnamic acid in a total amount of 2.0 to 10.0 wt%.

9. Use according to any one of claims 6 to 8, wherein the topically acceptable carrier is an oil or water and oil.

10. Use according to any one of claims 6 to 9, wherein the topical antiperspirant composition further comprises a lubricant.

11. Use according to any one of claims 6 to 10, wherein the topical antiperspirant composition further comprises a fragrance.

12. Use according to any one of claims 6 to 11, wherein the topical antiperspirant composition further comprises a thickener.

13. Use according to any one of claims 6 to 12, wherein the topical antiperspirant composition further comprises a preservative.

## Patentansprüche

1. Verwendung von *trans*-Zimtsäure als Antitranspirationsmittel zur Reduzierung oder Verhinderung von Schweiß.

2. Verwendung nach Anspruch 1 als Kosmetikum für die Körperpflege.

3. Verwendung nach Anspruch 1 oder Anspruch 2 zur topischen Anwendung auf der Haut.

4. Verwendung nach einem der Ansprüche 1 bis 3 zum Auftragen auf einen Hautbereich mit Schweißdrüsen.

5. Verwendung nach einem der Ansprüche 1 bis 4, wobei die *trans*-Zimtsäure als Antitranspirationsmittel und als Deodorant verwendet wird.

6. Verwendung nach einem der Ansprüche 1 bis 5, wobei eine topische Antitranspirationszusammensetzung das Antitranspirationsmittel umfasst, wobei die topische Antitranspirationszusammensetzung Folgendes umfasst:
(a) das Antitranspirationsmittel in einer Gesamtmenge von 1,5 bis 25,0 Gew.-% und
(b) einen topisch unbedenklichen Träger; und
wobei die Antitranspirationszusammensetzung in Form einer Creme, eines Gels, einer Paste, einer Flüssigkeit, einer Lotion, eines Schaums, eines Peelings oder eines Pulvers vorliegt.

7. Verwendung nach Anspruch 6, wobei die topische Antitranspirationszusammensetzung die *trans*-Zimtsäure in einer Gesamtmenge von 3,0 bis 20,0 Gew.-% umfasst.

8. Verwendung nach Anspruch 6, wobei die topische Antitranspirationszusammensetzung die *trans*-Zimtsäure in einer Gesamtmenge von 2,0 bis 10,0 Gew.-% umfasst.

9. Verwendung nach einem der Ansprüche 6 bis 8, wobei der topisch unbedenkliche Träger ein Öl oder Wasser und Öl ist.

10. Verwendung nach einem der Ansprüche 6 bis 9, wobei die topische Antitranspirationszusammensetzung ferner ein Gleitmittel umfasst.

11. Verwendung nach einem der Ansprüche 6 bis 10, wobei die topische Antitranspirationszusammensetzung ferner einen Duftstoff umfasst.

12. Verwendung nach einem der Ansprüche 6 bis 11, wobei die topische Antitranspirationszusammensetzung ferner ein Verdickungsmittel umfasst.

13. Verwendung nach einem der Ansprüche 6 bis 12, wobei die topische Antitranspirationszusammensetzung ferner ein Konservierungsmittel umfasst.

## Revendications

1. Utilisation de l'acide *trans*-cinnamique en tant qu'agent anti-transpirant pour réduire ou prévenir la transpiration.

2. Utilisation selon la revendication 1 en tant que produit cosmétique pour les soins personnels.

3. Utilisation selon la revendication 1 ou la revendication 2 pour une application topique sur la peau.

4. Utilisation selon l'une quelconque des revendications 1 à 3, pour une application sur une zone de peau comportant des glandes sudoripares.

5. Utilisation selon l'une quelconque des revendications 1 à 4, dans laquelle l'acide *trans*-cinnamique est utilisé en tant qu'agent anti-transpirant et en tant que déodorant.

6. Utilisation selon l'une quelconque des revendications 1 à 5, dans laquelle une composition anti-transpirante topique comprend l'agent anti-transpirant, dans laquelle la composition anti-transpirante topique comprend :
(a) l'agent anti-transpirant en une quantité totale de 1,5 à 25,0 % en poids, et
(b) un support acceptable pour une application topique ; et
dans laquelle la composition anti-transpirante se présente sous la forme d'une crème, d'un gel, d'une pâte, d'un liquide, d'une lotion, d'une mousse, d'un gommage ou d'une poudre.

7. Utilisation selon la revendication 6, dans laquelle la composition anti-transpirante topique comprend l'acide *trans*-cinnamique en une quantité totale de 3,0 à 20,0 % en poids.

8. Utilisation selon la revendication 6, dans laquelle la composition anti-transpirante topique comprend l'acide *trans*-cinnamique en une quantité totale de 2,0 à 10,0 % en poids.

9. Utilisation selon l'une quelconque des revendications 6 à 8, dans laquelle le support acceptable pour une application topique est une huile ou de l'eau et de l'huile.

10. Utilisation selon l'une quelconque des revendications 6 à 9, dans laquelle la composition anti-transpirante topique comprend en outre un lubrifiant.

11. Utilisation selon l'une quelconque des revendications 6 à 10, dans laquelle la composition anti-transpirante topique comprend en outre un parfum.

12. Utilisation selon l'une quelconque des revendications 6 à 11, dans laquelle la composition anti-transpirante topique comprend en outre un épaississant.

13. Utilisation selon l'une quelconque des revendications 6 à 12, dans laquelle la composition anti-transpirante topique comprend en outre un conservateur.
